(19) Europäisches Patentamt
European Patent Office
Office européen des brevets

(11) Veröffentlichungsnummer: **0 001 641**
**B1**

# (12) EUROPÄISCHE PATENTSCHRIFT

(45) Veröffentlichungstag der Patentschrift: **18.11.81**

(51) Int. Cl.³: **C 07 C 79/35**, A 01 N 39/02, C 07 C 153/09

(21) Anmeldenummer: **78101209.1**

(22) Anmeldetag: **24.10.78**

(54) **2-(2'-Nitro-5-(2''-chlor-4''-trifluormethylphenoxy)-phenoxy)-propionsäure-methoxyäthylester, ein diesen Ester als Wirkstoff enthaltendes Mittel und dessen Verwendung als Herbizid.**

(30) Priorität: **25.10.77 CH 12961/77**

(43) Veröffentlichungstag der Anmeldung: **02.05.79 Patentblatt 79/9**

(45) Bekanntmachung des Hinweises auf die Patenterteilung: **18.11.81 Patentblatt 81/46**

(84) Benannte Vertragsstaaten: **BE CH DE FR GB NL**

(56) Entgegenhaltungen:
**DE-A-2 628 384**
**DE-A-2 632 581**
**DE-A-2 639 796**
**DE-A-2 643 438**
**US-A-4 046 798**
**US-A-4 059 435**

(73) Patentinhaber: **CIBA-GEIGY AG, Patentabteilung Postfach, CH-4002 Basel (CH)**

(72) Erfinder: **Rohr, Otto, Dr., Kilbertweg 19, CH-4106 Therwil (CH)**
Erfinder: **Pissiotas, Georg, Dr., Breslauerstrasse 8, D-7850 Lörrach (DE)**
Erfinder: **Böhner, Beat, Dr., Hügelweg 3, CH-4102 Binningen (CH)**

(74) Vertreter: **Zumstein sen., Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# 2-[2'-Nitro-5-(2''-chlor-4''-trifluormethylphenoxy)-phenoxy]-propionsäure-methoxyäthylester, ein diesen Ester als Wirkstoff enthaltendes Mittel und dessen Verwendung als Herbizid.

Die vorliegende Erfindung betrifft den neuen herbizid wirksamen 2-[2'-Nitro-(2''-chlor-4''-trifluormethylphenoxy)-phenoxy]-propionsäure-methoxyäthylester, ein Mittel, welches diesen Ester als Wirkstoff enthält, sowie dessen Verwendung zur selektiven Bekämpfung von Unkräutern in Kulturen von Nutzpflanzen.

Der 2-[2'-Nitro-5- (2''-chlor- 4''-trifluormethylphenoxy)-phenoxy]-methoxyäthylester entspricht der Formel I,

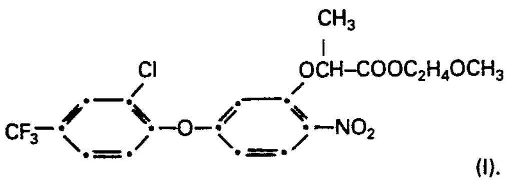

(I).

Herbizide Wirkstoffe auf der Basis von substituierten Diphenyläthern sind aus verschiedenen Patentschriften bekannt, so sind in der DE-A-2 311 638 und der schweizerischen Patentschrift No. 424 326 herbizid wirksame Diphenyläther beschrieben. Diese Verbindungen zeigen entweder eine ausserordentlich starke herbizide Wirkung (DE-A-2 311 638) bei geringer Selektivität oder einseitiger Wirkung, zum Beispiel nur gegen dikotyle Unkräuter.

Der neue Wirkstoff der Formel I gemäss vorliegender Erfindung besitzt eine allgemeine, nicht spezifisch gegen Gräser gerichtete Herbizidwirkung, vor allem bei post-emergenter Anwendung und kann in mono- und dikotylen Kulturen als Unkrautmittel eingesetzt werden. Überraschenderweise zeigt er nicht nur eine ausgezeichnete Wirkung gegen monokotyle und dikotyle Unkräuter, sondern auch eine ausgeprägte Selektivität in Gerste, Weizen und Reis, vor allem bei der post-emergenten Applikation.

Der Phenoxy-phenoxy-alkancarbonsäureester vorliegender Erfindung ist wenig giftig für Warmblüter und seine Anwendung dürfte keine Probleme verursachen. Die vorgeschlagenen Aufwandmengen liegen zwischen 0,1 und 5 kg pro Hektar.

Die Herstellung der neuen Verbindung der Formel I erfolgt nach an sich bekannten Reaktionen der chemischen Synthese.

Gemäss einem ersten Verfahren kann der 2-[2'-Nitro-5'-(2''-chlor-5''-trifluormethylphenoxy)-phenoxy]-methoxyäthylester hergestellt werden, indem man den 2-[2'-Nitro-(2''-chlor-5''-trifluormethylphenoxy)-phenoxy]-methoxyäthylester der Formel II nitriert

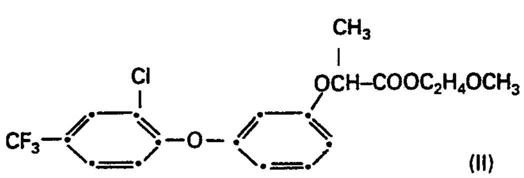

(II)

Die Nitrierung wird mittels Salpetersäure und Schwefelsäure bei tiefer Temperatur, −20 °C bis +20 °C in einem inerten organischen Lösungsmittel, z.B. einem chlorierten Kohlenwasserstoff vorgenommen. Es wird nur wenig mehr als die äquimolare Menge Salpetersäure verwendet. Auf diese Weise gelingt es, die Nitrogruppe fast ausschliesslich in die gewünschte ortho-Position zum Oxycarbonsäureesterrest zu bringen.

Der Ausgangsstoff der Formel II kann z.B. gemäss der DE-A-2 639 796 oder 2 732 442 resp. dem Schweiz. Patentgesuch Nr. 9321/76 hergestellt werden, durch Kondensation des 3,4-Dichlortrifluormethylbenzols mit dem O-(3-Hydroxyphenoxy)-milchsäure-methoxyäthylester.

Nach einem zweiten Verfahren kann man den 2-[2'-Nitro-5'-(2''-chlor-4''-trifluormethylphenoxy)-phenoxy]-methoxyäthylester der Formel I herstellen, indem man im 3-(2'-Chlor-4'-trifluormethylphenoxy)-phenol der Formel III

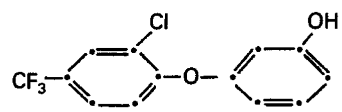

(III)

die freie Hydroxylgruppe mit einer Schutzgruppe versieht, z.B. durch Verestern mit einem Acylhalogenid (Acetylchlorid) in Gegenwart einer Base und dann den so acetylierten Diphenyläther bei tiefer Temperatur in einem inerten organischen Lösungsmittel mit der äquimolaren Menge Salpetersäure in Gegenwart von Schwefelsäure nitriert. Der erhaltene nitrierte und acylierte Diphenyläther wird dann in basischem Milieu verseift zum 2-Nitro-5-(2'-chlor-4'-trifluor-methylphenoxy)-phenol der Formel IV

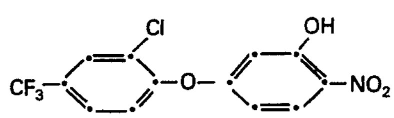

(IV),

der mittels eines α-Propionsäuremethoxyäthylesters der Formel V

$CH_3$
|
$Hal-CH-COOC_2H_4OCH_3$ (V),

worin Hal Chlor oder Brom bedeutet, in Gegenwart eines säurebindenden Mittels umgesetzt werden kann.

Die Reaktionspartner werden in diesen Verfahrensstufen in möglichst stöchiometrischen Mengen eingesetzt. Die Reaktionen erfolgen vorteilhafterweise in Anwesenheit von organischen, den Reaktionspartnern gegenüber inerten Lösungsmitteln. Bei den Nitrierungsstufen sollte die Temperatur möglichst tief gehalten werden. Bei den

Verseifungs- und Kondensationsstufen ist ein Temperaturbereich von Raumtemperatur bis Siedepunkt des Lösungsmittelgemisches angezeigt.

Bei gewissen Kondensationsstufen, in denen ein Halogenatom weggeht bzw. Halogenwasserstoffsäure gebildet wird, sollte die entsprechende Menge eines säurebindenden Mittels in die Reaktion gegeben werden.

Nach Beendigung der Reaktion wird das Endprodukt isoliert, z.B. durch Abdestillieren des Lösungsmittels und Eingiessen des Rückstandes in Eiswasser.

Nicht beschriebene Ausgangsstoffe lassen sich gemäss üblichen Verfahren und Techniken herstellen. Der Phenoxy-phenol kann beispielsweise gemäss der im J. Am. Chem. Soc. 61, 2702 (1939) oder in Chem. Abst. 54, 922h (1960) beschriebenen Methoden aus Resorzinmonoalkyläther und 3,4-Dichlor-trifluormethylbenzol hergestellt werden.

Schliesslich kann man den 2-[2'-Nitro-5'-(2''-chlor-4''-trifluormethylphenoxy)-phenoxy]-propionsäure-methoxyäthylester auch herstellen, indem man ein 2-[2'-Nitro-5'-(2''-chlor-4''-trifluormethylphenoxy)-phenoxy]-propionsäurehalogenid der Formel VI,

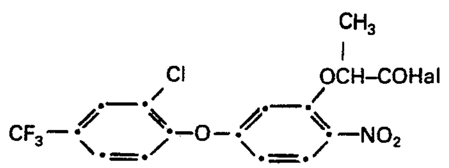

(VI),

worin Hal für Chlor oder Brom steht, mit 2-Methoxyäthanol umsetzt, gegebenenfalls in einem inerten organischen Lösungsmittel, in Gegenwart eines säurebindenden Mittels.

Der Ausgangsstoff der Formel VI kann am besten durch Verseifen eines schon bestehenden Esters zur 2-[2'-Nitro-5-(2''-chlor-4''-trifluormethylphenoxy)-phenoxy]-propionsäure erfolgen, welche dann mit einem Halogenierungsmittel wie Thionylchlorid, Thionylbromid, Phosphoroxychlorid oder -bromid, Phosphorpentachlorid- oder bromid, Sulfonylchlorid- oder Bromid zum Säurehalogen umgesetzt werden.

Als Lösungsmittel kommen fast alle organischen Lösungen in Frage, die die Reaktionspartner auflösen, aber nicht mit ihnen reagieren, wie z.B. Äther, Ketone, gewisse stabile Ester, flüssige Kohlenwasserstoffe aliphatische wie aromatische sowie chlorierte Kohlenwasserstoffe. Als säurebindende Mittel werden vorzugsweise Trialkylamine eingesetzt, sofern es die Löslichkeit ermöglicht, jedoch auch Alkalimetallhydroxyde, Carbonate oder Ammoniaklösungen.

Das nachfolgende Beispiel veranschaulicht die Herstellung des Esters der Formel I.

Beispiel

2-[2-Nitro-5-(2'-chlor-4'-trifluormethylphenoxy)-phenoxy]-propionsäure-methoxyäthyl-ester

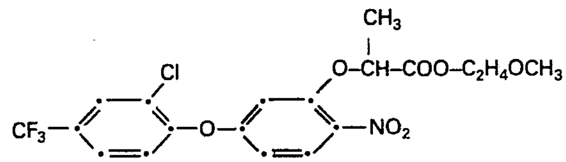

Zu einer Lösung von 42,5 g (α-[2-Nitro-3-(2'-chlor-4'-trifluormethylphenoxy)-phenoxy]-propionsäurechlorid und 20 g Methoxyäthanol in 150 ml Toluol tropft man bei 5 °–15 °C 20 g Triäthylamin. Man rührt über Nacht bei Raumtemperatur weiter, versetzt anschliessend das Reaktionsgemisch mit 50 ml Wasser und trennt die organische Phase ab. Diese wird über Natriumsulfat getrocknet und das Lösungsmittel abdestilliert. Durch Destillation im Hochvakuum erhält man 20,8 g eines zähflüssigen Öls mit einem Siedebereich von 200–210 °C bei 0.04 Millibar.

Die Erfindung betrifft auch herbizide und pflanzenwachstumsregulierende Mittel, welche den neuen Wirkstoff der Formel I enthalten, sowie Verfahren zur pre- und post-emergenten Unkrautbekämpfung.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

| | |
|---|---|
| feste Aufarbeitungsformen: | Stäubemittel, Streumittel, Granulate, Umhüllungsgranulate, Imprägnierungsgranulate und Homogengranulate; |
| in Wasser dispergierbare Wirkstoffkonzentrate: | Spritzpulver, (wettable powder), Pasten, Emulsionen: |
| flüssige Aufarbeitungsformen: | Lösungen. |

Stäubemittel

Zur Herstellung eines a) 5%igen und b) 2%igen Stäubemittels werden die folgenden Stoffe verwendet:

| | | | |
|---|---|---|---|
| a) | 5 Teile | 2-[2-Nitro-5-(2'-chlor-4'-trifluormethyl-phenoxy)-phenoxy]-propionsäure-methoxyäthylester, |
| | 95 Teile | Talkum |
| b) | 2 Teile | des obigen Wirkstoffes |
| | 1 Teil | hochdisperse Kieselsäure, |
| | 97 Teile | Talkum; |

Die Wirkstoffe werden mit den Trägerstoffen vermischt und vermahlen.

Granulate

Zur Herstellung eines 5%igen Granulates werden die folgenden Stoffe verwendet:

| | | |
|---|---|---|
| 5 | Teile | des obigen Wirkstoffes |
| 0,25 | Teile | Epichlorhydrin |
| 0,25 | Teile | Cetylpolyäthylenglykoläther mit 8 Mol Äthylenoxid, |
| 3,50 | Teile | Polyäthylenglykol, |
| 91 | Teile | Kaolin (Korngrösse 0,3 bis 0,8 mm) |

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum verdampft.

Der Gehalt an Wirkstoff in den oben beschriebenen Mitteln liegt zwischen 0,1 bis 95%, bevorzugt zwischen 1 bis 80%. Anwendungsformen können bis hinab zu 0,001% verdünnt werden. Die Aufwandmengen betragen in der Regel 0,1 bis 10 kg AS/ha, vorzugsweise 0,25 bis 5 kg AS/ha. Die Wirkstoffe der Formel I können beispielsweise wie folgt formuliert werden (Teile bedeuten Gewichtsteile):

Emulsionskonzentrat

Zur Herstellung eines 25%igen Emulsionskonzentrates werden

| | |
|---|---|
| 25 Teile | 2-[2-Nitro-5-(2'-chlor-4'-trifluormethylphenoxy)phenoxy]-propionsäure-methoxyäthylester, |
| 5 Teile | Mischung von Nonylphenolpolyoxyäthylen und Calcium-dodecylbenzol-sulfonat, |
| 15 Teile | Cyclohexanon, |
| 55 Teile | Xylol |

miteinander vermischt. Dieses Konzentrat kann mit Wasser zu Emulsionen auf geeignete Konzentrationen verdünnt werden.

Spritzpulver

Zur Herstellung eines a) 50%igen, b) 25%igen und c) 10%igen Spritzpulvers werden folgende Bestandteile verwendet:

| | | |
|---|---|---|
| a) | 50 Teile | 2-[2-Nitro-5-(4'-trifluormethylphenoxy)-phenoxy]-propionsäure-methoxyäthylester, |
| | 5 Teile | Natriumdibutylnaphthylsulfonat, |
| | 3 Teile | Naphthalinsulfonsäuren-Phenonolsulfonsäuren-Formaldehyd-Kondensat 3:2:1, |
| | 20 Teile | Kaolin, |
| | 22 Teile | Champagne-Kreide; |
| b) | 25 Teile | des obigen Wirkstoffes, |
| | 5 Teile | Oleylmethyltaurid-Natrium-Salz, |
| | 2,5 Teile | Naphthalinsulfonsäuren-Formaldehyd-Kondensat, |
| | 0,5 Teile | Carboxymethylcellulose, |
| | 5 Teile | neutrales Kalium-Aluminium-Silikat, |
| | 62 Teile | Kaolin; |
| c) | 10 Teile | des obigen Wirkstoffs, |
| | 3 Teile | Gemisch der Natriumsalze von gesättigten Fettalkoholen, |
| | 5 Teile | Naphthalinsulfonsäuren-Formaldehyd-Kondensat |
| | 82 Teile | Kaolin. |

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden.

Die Verbindung der Formel I besitzt auch günstige wachstumsregulierende Effekte. Sie eignet sich zur Defoliation und Desiccation oberirdischer unverholzter Pflanzenteile, insbesondere zur Defoliation und Desiccation vom Bauwollpflanzen, Leguminosen, Sorghum, Soja, Kartoffeln und Reben vor der Ernte. Diese Wirkung wurde wie folgt geprüft:

Defoliation und Dessication

Baumwollpflanzen der Sorte «Delta Pine» werden im Gewächshaus gezogen. Nach ihrer Blüte werden sie mit einer Wirkstoff-Suspension so gespritzt, dass Wirkstoff in Mengen aufgetragen wird, welche einer Aufwandmenge von 1.2, 0.6 und 0.3 kg pro Hektar im Feld entspräche. Die Pflanzen werden dann im Gewächshaus gelassen und der Versuch 15 Tage nach der Behandlung ausgewertet.

Defoliation

Die Blätter werden vor der Behandlung und bei der Auswertung des Versuches gezählt. Der Verlust wird notiert und gemäss dem untenstehenden Schema in Zahlen ausgedrückt.

Dessication

Die an der Pflanze verbleibenden Blätter werden auf den Zustand ihrer Austrocknung hin ausgewertet und das Resultat ebenfalls wie unten angegeben in Zahlen ausgedrückt:

| | |
|---|---|
| 9 = | 0– 11% Blattfall oder Austrocknung |
| 8 = | 11– 22% Blattfall oder Austrocknung |
| 7 = | 23– 33% Blattfall oder Austrocknung etc. |
| 2 = | 78– 88% Blattfall oder Austrocknung |
| 1 = | 89–100% Blattfall oder Austrocknung |

Paste

Zur Herstellung einer 45%igen Paste werden folgende Stoffe verwendet:

| | |
|---|---|
| 45 Teile | 2-[2-Nitro-5-(2'-chlor-4'-trifluormethyl-phenoxy)-phenoxy]-propionsäure-methoxyäthylester |
| 5 Teile | Natriumaluminiumsilikat, |
| 14 Teile | Cetylpolyäthylenglykoläther mit 8 Mol Äthylenoxid, |
| 1 Teil | Oleylpolyäthylenglykoläther mit 5 Mol Äthylenoxid, |
| 2 Teile | Spindelöl, |
| 23 Teile | Wasser, |
| 10 Teile | Polyäthylenglykol. |

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Der in den erfindungsgemässen Mitteln enthaltene Wirkstoff beeinflusst das Pflanzenwachstum in verschiedener Weise. So hemmt, verzögert oder unterbindet er in erster Linie das Wachstum und die Keimung. Es handelt sich dabei also sowohl um pre- und postemergente Herbizidwirkung als auch um Wuchshemmung.

Der neue Wirkstoff der Formel I hat eine gute Wirkung gegen monokotyle und dikotyle Unkräuter, sowie eine ausgeprägte Selektivität in Gerste, Weizen und Reis vor allem bei der postemergenten Applikation.

Zum Nachweis der Brauchbarkeit als Herbizide (pre- und post-emergent) dienten folgende Testmethoden:

Pre-emergente Herbizid-Wirkung (Keimhemmung)

Im Gewächshaus wird unmittelbar nach der Einsaat der Versuchspflanzen in Saatschalen die Erdoberfläche mit einer wässerigen Suspension des Wirkstoffs, erhalten aus einem 25%-igen Spritzpulver, behandelt. Es wurden verschiedene Konzentrationsreihen angewendet, entsprechend 4 und 2 kg Wirksubstanz pro Hektar. Die Saatschalen werden im Gewächshaus bei 22–25 °C und 50–70% rel. Luftfeuchtigkeit gehalten und der Versuch nach 3 Wochen ausgewertet und die Resultate nach folgender Notenskale bonitiert:

| | |
|---|---|
| 1 = | Pflanzen nicht gekeimt oder total abgestorben |
| 2–8 = | Zwischenstufen der Schädigung |
| 9 = | Pflanzen ungeschädigt (wie unbehandelte Kontrolle). |

Post-emergente Herbizid-Wirkung (Kontaktherbizid)

Eine grössere Anzahl (mindestens 7) Unkräuter und Kulturpflanzen, sowohl monocotyle wie dicotyle, wurden nach dem Auflaufen (im 4-bis-6-Blattstadium) mit einer wässrigen Wirkstoffemulsion in Dosierungen von 0,5 und 1 kg Wirksubstanz pro Hektar auf die Pflanzen gespritzt und diese bei 24 °–26 °C und 45–60% rel. Luftfeuchtigkeit gehalten. 15 Tage nach der Behandlung wird der Versuch ausgewertet und das Ergebnis wie im preemergent-Versuch nach derselben Notenskala bonitiert.

Vorauflauf (pre-emergent)

| Verbindung No. | I | | A | |
|---|---|---|---|---|
| Aufwandmenge kg/ha | 4 | 2 | 4 | 2 |
| Pflanze | | | | |
| Gerste | 6 | 8 | 1 | 1 |
| Weizen | 9 | 9 | 1 | 1 |
| Mais | 2 | 4 | 2 | 2 |
| avena fatua | 6 | 8 | 1 | 1 |
| lolium perenne | 6 | 9 | 1 | 1 |
| alopecurus myos. | 3 | 6 | 1 | 1 |
| cyperus esc. | 2 | 3 | 4 | 9 |
| rottboellia exalt. | 3 | 7 | 1 | 1 |
| digitaria sang. | 1 | 1 | 1 | 1 |
| setaria italica | 1 | 1 | 1 | 1 |
| echinochloa c.g. | 1 | 1 | 1 | 1 |
| sida spinosa | 1 | 1 | 1 | 1 |
| sesbania exaltata | 1 | 4 | 1 | 1 |
| amaranthus retrof. | 1 | 1 | 1 | 1 |
| sinapis alba | 1 | 1 | 1 | 3 |
| ipomoea purp. | 1 | 1 | 1 | 1 |
| galium aparine | 1 | 1 | 1 | 1 |
| pastinaca sativa | 1 | 1 | 1 | 1 |
| rumex sp. | 1 | 1 | 1 | 1 |
| chrysanthemum 1. | 1 | 1 | 1 | 1 |
| abutilon sp. | 1 | 1 | 1 | 1 |
| solanum nigrum | 1 | 1 | 1 | 1 |

A = 2-Chlor-4-Trifluormethyl-3'-äthoxy-4'-nitro-di-phenyläther, bekannt aus der DE-A-2 311 638 (Ver-bindung 3 Z.)

Nachauflauf (post-emergent)

| Verbindung No. | I | | A | |
|---|---|---|---|---|
| Aufwandmenge kg/ha | 1 | 0.5 | 1 | 0.5 |
| Pflanze | | | | |
| Gerste | 4 | 7 | 1 | 1 |
| Weizen | 6 | 8 | 2 | 2 |
| Reis | 3 | 8 | 1 | 1 |
| avena fatua | 3 | 7 | 1 | 1 |
| lolium perenne | 3 | 6 | 1 | 1 |
| alopecurus myos. | 2 | 2 | 1 | 1 |
| cyperus esc. | 3 | 3 | 3 | 3 |
| rottboellia exalt. | 2 | 4 | 1 | 2 |
| digitaria sang. | 1 | 2 | 1 | 1 |
| setaria italica | 1 | 1 | 1 | 1 |
| echinochloa c.g. | 1 | 2 | 1 | 1 |
| sida spinosa | 1 | 2 | 2 | 2 |
| sesbania exalt. | 1 | 1 | 1 | 1 |
| amaranthus retro. | 1 | 1 | 1 | 1 |
| sinapis alba | 1 | 1 | 1 | 1 |
| ipomoea purp. | 1 | 1 | 1 | 1 |
| galium aparine | 1 | 1 | 1 | 1 |
| pastinaca sativa | 1 | 1 | 1 | 1 |
| chrysanthemum leuc. | 1 | 1 | 4 | 4 |
| abutilon sp. | 1 | 1 | 1 | 1 |
| solanum nigrum | 1 | 1 | 1 | 1 |
| matricaria cham. | 1 | 1 | 2 | 2 |

| Verbindung | Defoliation | | (Dessication) |
|---|---|---|---|
| Aufwandmenge in kg/ha | 1,2 | 0,6 | 0,3 |
| I | 2(1) | 2(1) | 3(1) |
| B | 4(1) | 7(2) | 7(3) |

B = 2-[2-Nitro-5-(2'-chlor-4'-trifluormethylphenoxy)-phenoxy]-propionsäuremethylester, bekannt aus DE-A-2 311 638 (Verbindung 32 Z).

**Patentansprüche**

1. 2-[2'-Nitro-5'-(2''-chlor-4''trifluormethylphenoxy)-phenoxy]-propionsäuremethoxyäthylester der Formel

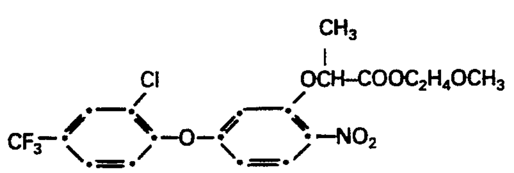

2. Herbizides Mittel, dadurch gekennzeichnet, dass es als Wirkstoff den 2-[2'-Nitro-5'-(2''-chlor-4''-trifluor-methyl-phenoxy)-phenoxy]-propionsäure-methoxyäthylester enthält.

3. Die Verwendung des 2-[2'-Nitro-5-'-(2''-chlor-4''-trifluormethylphenoxy)-phenoxy]-propionsäure-methoxyäthylesters oder des ihn enthaltenden Mittels zur selektiven Bekämpfung von Unkräutern in Kulturen von Nutzpflanzen, vor allem Getreide und Reis.

4. Die Verwendung des 2-[2'-Nitro-5'-(2''-chlor-4''-trifluormethylphenoxy)-phenoxy]-propionsäure-methoxyäthylesters oder des ihn enthaltenden Mittels zur Desikkation und Defoliation von Baumwolle- oder Kartoffelkulturen kurz vor deren Ernte.

**Revendications**

1. Le 2-[2'-nitro-5'-(2''-chloro-4''-trifluorométhylphénoxy)-phénoxy]-propionate de méthoxyéthyle, de formule

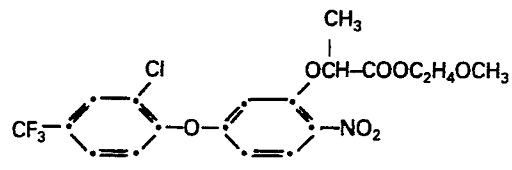

2. Produit herbicide caractérisé en ce qu'il contient en tant que substance active le 2-[2'-nitro-5'-(2''-chloro-4''-trifluorométhylphénoxy)-phénoxy]-propionate de méthoxyéthyle.

3. L'utilisation du 2-[2'-nitro-5'-(2''-chloro-4''-trifluorométhylphénoxy)-phénoxy]-propionate de méthoxyéthyle ou du produit le contenant pour la lutte sélective contre les mauvaises herbes dans les cultures de végétaux utiles, surtout de céréales et de riz.

4. L'utilisation du 2-[2'-nitro-5'-(2''-chloro-4''tri-fluorométhylphénoxy)-phénoxy]-propionate de méthoxyéthyle ou du produit le contenant pour la dessiccation et la défoliation de cultures de coton ou de pommes de terre peu avant la récolte.

**Claims**

1. A 2-[2'-nitro-5'-(2''-chloro-4''-trifluoromethylphenoxy)-phenoxy]-propionic acid methoxyethyl ester of the formula

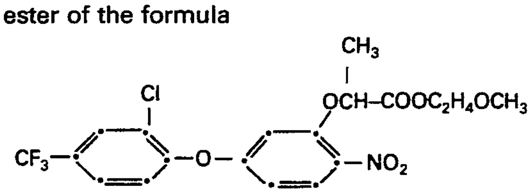

2. A herbicidal composition, characterised in that it contains, as active ingredient, a 2-[2'-nitro-5'-(2''-chloro-4''-trifluoromethylphenoxy)-phenoxy]-propionic acid methoxyethyl ester.

3. A method of selectively controlling weeds in crops of useful platns, especially cereals and rice, which comprises the use of a 2-[2'-nitro-5'-(2''-chloro-4''-trifluoromethyl-phenoxy)-phenoxy]-propionic acid methoxyethyl ester or of a composition containing such a compound.

4. A method of desiccating and defoliating cotton or potato plants shortly before harvesting, which comprises the use of a 2-[2'-nitro-5'-(2''-chloro-4''-trifluoromethyl-phenoxy)-phenoxy]-propionic acid methoxyethyl ester or of a composition containing such a compound.